Europäisches Patentamt

European Patent Office

Office européen des brevets

(11) Veröffentlichungsnummer: **0 129 646**

**A1**

(12) # EUROPÄISCHE PATENTANMELDUNG

(21) Anmeldenummer: 84103421.8

(22) Anmeldetag: 28.03.84

(51) Int. Cl.⁴: **G 01 N 33/96**
**G 01 N 33/72**

(30) Priorität: 29.03.83 DE 3311458

(43) Veröffentlichungstag der Anmeldung:
02.01.85 Patentblatt 85/1

(84) Benannte Vertragsstaaten:
BE FR GB IT NL SE

(71) Anmelder: PANCHEM Gesellschaft für chemische
Produkte mbH
Schlossstrasse 3
D-8751 Kleinwallstadt(DE)

(72) Erfinder: Gain, Thomas
Oberföhringerstrasse 129
D-8000 München 81(DE)

(74) Vertreter: Wuesthoff, Franz, Dr.-Ing. et al,
Patentanwälte Wuesthoff -v. Pechmann-Behrens-Goetz
Schweigerstrasse 2
D-8000 München 90(DE)

(54) Verfahren zur Herstellung eines glukosefreien Hämoglobinkontrollmittels für die HbAI-Bestimmung.

(57) Bei einem Verfahren zur Herstellung eines glukosefreien Kontrollmittels zur Bestimmung von glykosiliertem Hämoglobin wird Hämoglobin mit seinem bekannten Gehalt an glykosiliertem Hämoglobin HbAₗₐ₊ᵦ₊c mit K₃(FeCN)₆/KCN-Lösung in Cyanmethämoglobin umgewandelt, mit einem Natrium-Phosphat-Cyanid-Puffer stabilisiert und durch Lyophilisieren bei -40°C und anschließender Temperaturanhebung unter Hochvakuum auf -20°C haltbar gemacht, bei dieser Temperatur unter Lichtausschluß aufbewahrt, wonach die so erhaltene glukosefreie glykosilierte Hämoglobinkontrolle durch Zugabe eines wässrigen Mediums rekonstituiert werden kann.

EP 0 129 646 A1

PATENTANWÄLTE

WUESTHOFF - v. PECHMANN - BEHRENS - GOETZ

EUROPEAN PATENT ATTORNEYS

DR.-ING. FRAY
DR. PHIL. Fh.
DIPL.-ING. G.
DIPL.-CHEM.
DR.-ING. DIE
DIPL.-ING.; D...

0129646

IMANN

.T GOETZ

D-8000 MÜNCHEN 90
SCHWEIGERSTRASSE 2

TELEFON: (089) 66 20 51
TELEGRAMM: PROTECTPATENT
TELEX: 5 24 070

panchem gmbh

Kleinwallstadt

BEZEICHNUNG GEÄNDERT
siehe Titelseite

### Verfahren zur Herstellung
### einer glukosefreien Hämoglobinkontrolle

Für die Stoffwechselkontrolle von Diabetikern hat sich die Bestimmung von glykosilierten Hämoglobinen in der Diabetesüberwachung neben den Blut- und Urinzuckerbestimmungen in der klinischen Praxis als aussagekräftiges und zuverlässiges Verfahren bewährt.

Hämoglobin, der Farbstoff der roten Blutkörperchen, besteht zu etwa 5 % aus einer Farbkomponente, dem Häm, und zu etwa 95 % aus einer Eiweißkomponente, dem Globin, die beide komplexartig miteinander verknüpft sind.

Es hat sich nun gezeigt, daß bei der säulenchromatographischen Auftrennung von Hämoglobinlösungen der Hauptanteil an Hämoglobin, nämlich $HbA_1$, inhomogen ist, d.h., vor der Hauptfraktion ließ sich eine Reihe schneller wandernder Hämoglobine, die als $HbA_1$ bezeichnet werden, eluieren. Diese Hämoglobinfraktion läßt sich in die Komponeten $HbA_{Ia}$, $HbA_{Ib}$ und $HbA_{Ic}$ aufspalten. Die chemische Strukturaufklärung zeigte, daß es sich dabei um Glykoproteine, d.h., um Glykosilierungsprodukte vom Hämoglobin $A_I$ handelt, die durch eine Kondensationsreaktion der Aldehydgruppe von Kohlenhydratmolekülen, wie Fruktose-1,6-Diphosphat, Glukose-6-Phosphat und Glukose mit endständigen Aminogruppen gebildet werden. Eine Gleichgewichtsreaktion führt zunächst zur Bildung der instabilen Aldiminform (Schiff'sche Base), die dann über eine Amadoriumlagerung irreversibel zum Ketoamin führt. Das glykosilierte Hämoglobin ($HbA_{Ia}$, $HbA_{Ib}$ und $HbA_{Ic}$) wird in einer nicht enzymatischen Reaktion während der Erythrozyten-Lebensdauer von ca. 120 Tagen gebildet.

Während bei Stoffwechsel-Gesunden-Nichtdiabetikern der Anteil an glykosilierten Hämoglobin zwischen 3,5 bis 7,5 % liegt, kann sich bei Diabetikern dieser Anteil auf 20 % und mehr bezogen auf das Gesamt-Hb erhöhen.

/2

**0129646**

Die US-PS 3 964 865 offenbart einen lyophilisierten Hämoglobinstandard für die kolorimetrische Bestimmung des Totalhämoglobins, der dadurch hergestellt wird, dass man eine "rohe" Hämoglobinfraktion filtriert, wobei durch bei der Filtration zugeführten Luftsauerstoff das Hämoglobin zu Methämoglobin, d.h. zu Oxymethämoglobin aufoxidiert wird. Das Methämoglobin wird nach Einstellen auf einen bestimmten gewünschten Konentrationswert lyophilisiert und eignet sich in dieser Form als Standard-Mittel für die kolorimetrische Bestimmung von Totalhämoglobin. Um den von der National Academy of Sciences - National Research Council, USA (NRC) und dem National Committee for Standardization in Haematology (ICSH) geforderten Normen für die Bestimmung des Hämoglobingewichts gerecht zu werden, wird der lyophilisierte Standard vor seinem Einsatz zu Cyanmethämoglobin umgewandelt.

Mit solch einem, aus dem Stand der Technik bekannten Standard ist jedoch nur eine spektroskopische Bestimmung von Totalhämoglobin, nicht jedoch eine Bestimmung einzelner Hämoglobinfraktionen wie z.B. der $HbA_I$-Fraktion, möglich, da bei der Aufoxidation des Hämoglobins zu Methämoglobin die prosthetischen Gruppen als auch das Ladungsverhalten der Ketten verändert werden, so dass solch ein Standard ein z.B. anderes chromatographisches Verhalten aufweist als Nativblut und daher weder für eine Direktbestimmung der Hämoglobinfraktionen noch als Kontroll-Mittel für die richtige Handhabung des für die z.B. $HbA_I$-Bestimmung gewählten Trennverfahrens eingesetzt werden kann.

/3

Für die Bestimmung des Prozentanteils glykosilierten Hämoglobins am Gesamthämoglobin stehen verschiedene Verfahren zur Verfügung. Aufgrund der stärker negativen Ladung der glykosilierten Hämoglobine $HbA_I$ lassen sich diese von anderen Hämoglobinen mit Hilfe der Ionenaustauschchromatographie, der Hochdruckflüssigkeitschromatographie, der Isofokussierung und der Elektroosmose abtrennen. Eine weitere Methode zur Bestimmung glykosilierter Hämoglobine beruht auf der Hydrolyse der glykosilierten Hämoglobine in Oxalsäure und Umwandlung der abgespaltenen Hexosen in 5-Hydroxymethylfurfural, das nach Reaktion mit Thiobarbitursäure (TBA) photometrisch erfaßbar ist. Ebenfalls ist eine affinitätschromatographische Abtrennung unter Ausnutzung der Hydroxylgruppen von den an das Hämglobin gebundenen Kohlenhydraten möglich.

Bei Anwendung der verschiedenen Bestimmungsmethoden tritt jedoch der Nachteil auf, daß die Analysenergebnisse ein und derselben Probe je nach angewandtem Verfahren unterschiedlich miteinander korrelieren. So sind beispielsweise unabhängig davon, ob Makro- oder Mikrosäule für die chromatographische Bestimmung des Prozentgehaltes an Glykohämoglobin verwendet werden, die mit der chemischen TBA-Methode gefundenen $HbA_I$-Werte nur über einen Korrekturfaktor vergleichbar. Zudem werden bei den gängigen Analyseverfahren zum Teil die Gesamtfraktion $HbA_I$ als auch die aufgetrennten Einzelfraktionen $HbA_{Ia,b}$ und $HbA_{Ic}$ erfaßt, so daß die Analysenwerte nicht einheitlich angegeben werden und daher ebenfalls nur schwer miteinander zu vergleichen sind. Bestimmt man beispielsweise die glykosilierten Hämglobinanteile $HbA_{Ia+b+c}$, die sowohl mit der konventionellen Makrosäulenchromatographie als auch mit der Hochdruckflüssigkeitschromatographie gut erfaßt werden können, so zeigt die Auswertung der Ergebnisse lediglich eine Korrelation, aber keine Übereinstimmung zwischen den beiden Verfahren.

Zusammenfassend kann gesagt werden, daß die einzelnen Methoden zur Bestimmung der glykosilierten Hämoglobine, insbesondere die Ionenaustauschchromatographie, hinsichtlich der Auftrennung der Fraktionen gute Ergebnisse liefern, diese jedoch nicht, je nach eingesetztem Verfahren, übertragbar und somit vergleichbar sind. Für die interne Kontrolle der spezifischen Trennverfahren werden bis heute Standardkontrollen verwendet, die jeweils nur für ein bestimmtes Verfahren eingesetzt werden können. Die Vergleichbarkeit aller angewandten Trenntechniken und damit der aufgefundenen Werte über eine Universalkontrolle ist jedoch eine wichtige Forderung für die klinischen Routineuntersuchungen geblieben.

/4

Der Erfindung liegt die Aufgabe zugrunde, ein Verfahren zur Herstellung einer stabilen glykosilierten Hämoglobinkontrolle zur Verfügung zu stellen, das eine Qualitätskontrolle der $HbA_I$-Bestimmung aller angewandter Trennverfahren intern und extern mit identischem Material ermöglicht.

Diese Aufgabe wird bei dem erfindungsgemäßen Verfahren durch die kennzeichnenden Merkmale des Patenanspruches 1 gelöst.

Das Verfahren zur Herstellung einer stabilen glykosilierten Hämoglobinkontrolle beruht auf der Umwandlung von Hämoglobin in Cyanmethämoglobin. Die erfindungsgemäß hergestellten Cyanmethämoglobine behalten auch bei längerer Aufbewahrung ein konstantes Extinktionsverhältnis bei, währenddessen die Extinktionsverhältnisse anders substituierter Hämoglobine bei längerer Lagerung nicht mehr Hb-Variantenspezifisch verbleiben und somit als Standardkontrollen nicht geeignet sind.

Für die erfolgreiche Herstellung der glykosilierten Hämoglobinstandards hat es sich nun als besonders vorteilhaft erwiesen, vor der Umwandlung des freien Hämoglobins/der glykosilierten Hämoglobine in die entsprechenden Cyanmethämoglobine, die Erythrozyten vor der Hämolyse mehrmals mit verdünnter isotoner NaCl-Lösung zu waschen und danach das Hämolysat mit einem Na-Phosphat-Cyanid-Puffer zu stabilisieren, da durch diese Maßnahme der unter den Reaktionsbedingungen sonst instabile $K_3\left[Fe\,(CN)_6\right]$-Komplex seine oxidierende Wirkung einerseits beibehält und andererseits keine unerwünschten Reaktionen an den Globinketten hervorruft. Anstelle des maskierenden CN-Ions läßt sich beispielsweise auch das SCN-Ion verwenden.

Zum sauberen Entfernen von Zellresten und Lipiden hat es sich als günstig erwiesen, vorteilhafterweise das Hämolysat anschließend mit $CCl_4$ zu behandeln. Anstelle von $CCl_4$ läßt sich beispielsweise auch ein anderes fettlösendes Mittel, wie Toluol usw., verwenden, das jedoch ansonsten gegenüber dem Hämoglobin inert sein muß.

Wenn die erfindungsgemäßen Hämoglobinkontrollen nicht sofort nach deren Herstellung zum Einsatz kommen, werden sie im Hochvakuum durch Gefrieren bei zunächst tiefen Temperaturen mit anschließender langsamer Temperaturerhöhung sehr schonend lyophilisiert. Diesem Vorgang kommt eine besondere Bedeutung zu, da der Eiweißanteil des Hämoglobins nicht denaturiert werden darf, um die Funktionsfähigkeit der Hämoglobinkontrolle als Qualitätskontrolle bei der $HbA_I$-

Bestimmung nicht zu gefährden. Derart hergestellte Qualitätskontrollen sind dann unter Lichtausschluß gekühlt mindestens für 8 Monate haltbar.

Im Hinblick auf die TBA-Methode ist es besonders hervorzuheben, daß die nach dem neu geschaffenen Verfahren hergestellten Glykohämoglobin-Kontrollen keine ungebundene Glukose enthalten, weil diese nicht mehr zur Stabilisierung des Äquilibriums notwendig ist und auch während ihrer Herstellung und Lagerung keine Spaltung der glykosilierten Hämoglobine in die entsprechenden Hexosen und Hämoglobine auftritt. Dies verhindert, daß die TBA-Methode aufgrund der sonst unspezifisch vermehrten 5-Hydroxymethyl-furfurol-Bildung erhöhte Werte liefert.

Damit die Löslichkeit der Hämoglobinkontrollen nicht durch einen Aussalzeffekt verringert wird und so bei einigen Methoden zu einer falschen Aussage betreffend der Hämoglobinanteile führt, wird erfindungsgemäß eine Entsalzung, z.B. durch Gelfiltration oder Dialyse, durchgeführt.

Durch Zugabe von wässrigen Medien lassen sich die lyophilisierten Hämoglobinkontrollen sehr schnell und einfach für die Verwendung als Qualitätskontrolle bei Bedarf rekonstituieren.

Das folgende Beispiel beschreibt das erfindungsgemäße Verfahren zur Herstellung einer stabilen, glukosefreien glykosilierten Hämoglobinkontrolle.

Beispiel

Zunächst werden 1 ml Erythrozyten mit 20 ml isotoner NaCl-Lösung dreimal gewaschen, wobei der Überstand nach dem Zentrifugieren jeweils abgesaugt wird. Zur Hämolyse wird dann 1 ml destilliertes Wasser beigefügt und für 2 Minuten stehengelassen. Die Umwandlung des Hämoglobins zu Cyanmethämoglobin erfolgt durch Zugabe von 80 $\mu$l einer 5 %igen $K_3\left[Fe(CN)_6\right]$-Lösung und 2 ml einer 0,5 %igen KCN-Lösung unter jeweiligem 2minütigem Stehenlassen und mehrmaligem kräftigen Mischen. Anschließend wird das Hämolysat mit 12 ml Na-Phosphat-Cyanid-Puffer bei ca. pH 6,7 verdünnt. Dieser pH-Wert wird üblicherweise erzielt, wenn man 4,59 g $NaH_2PO_4$ x $H_2O$ sowie 1,18 g $Na_2HPO_4$ und 0,65 g KCN mit destilliertem Wasser in einem Gesamtvolumen von 1 l löst. Zur Entfernung von Lipiden und Zellresten werden anschließend 2 ml $CCl_4$ zugegeben und 10 Minuten zentrifugiert. Anschließend wird nach üblichen Verfahrensweisen, z.B. durch Gelfiltration oder Dialyse, eine Entsalzung durchgeführt.

0129646

Der oben genannte Ansatz ergibt ca. 24 Kontrollstandards. Zur Haltbarmachung der Kontrollstandards werden diese im Hochvakuum bei -40°C und anschließender langsamer Temperaturerhöhung während 48 Stunden auf -20°C lyophilisiert.

Es hat sich gezeigt, daß die Haltbarkeit bei Aufbewahrung der Standardkontrollen unter Kühlung im Dunklen mindestens 8 Monate beträgt. Der Einsatz der Qualitätskontrollen erfolgt nach Rekonstituierung der Kontrollstandards durch Zugabe von 0,5 bzw. 0,2 ml vorzugsweise destilliertem Wasser. Der Gesamthämoglobingehalt des Kontrollstandards liegt bei oben genanntem Beispiel zwischen 4 bis 6 mg pro 0,5 ml. Die exakte Ermittlung erfolgt auf übliche Weise.

Die nach dem erfindungsgemäßen Verfahren hergestellten Standardkontrollen können für alle Bestimmungsmethoden glykosilierter Hämoglobine unter Berücksichtigung ihrer spezifischen Nachweismethoden und Normbereiche verwendet werden, so daß erstmalig eine Universalkontrolle für die Bestimmung der $HbA_1$-Fraktion des Hämoglobins zur Verfügung steht.

Dies wird in nachfolgender Tabelle 1 veranschaulicht.

Tabelle 1

Bestimmung von glykosilierten Hämoglobinen mittels verschiedener Trenntechniken unter Einsatz einer erfindungsgemäßen Hämoglobinkontrolle identischer Charge bei 10-fach-Bestimmungen.

(Die angegebenen Prozentzahlen der jeweiligen glykosilierten Hämoglobinfraktionen sind relative Prozentwerte, bezogen auf das Gesamthämoglobin.)

| Bestimmungsmethode | % $HbA_{Ia+b}$ | % $HbA_{Ic}$ | % $HbA_{Iges.}$ = % $HbA_{Ia+b}$ + $HbA_{Ic}$ |
|---|---|---|---|
| Makrosäule | 3,8 | 6,9 | 10,7 |
| Hochdruckflüssig-keitschromatographie (HPLC) | 3,6 | 6,7 | 10,3 |
| TBA | | 6,4 | |
| Affinitäts-chromatographie | | | 11,3 |
| Mikrosäule | | | 9,8 |
| Elektroosmose | | | 7,3 |
| Isofokussierung | 2,8 | 4,2 | 7,0 |

Wie aus Tabelle 1 zu entnehmen ist, liefert der Einsatz der erfindungsgemäßen Hämoglobinkontrollen bei unterschiedlichen, jedoch vergleichbaren Trenntechniken, wie der Hochdruckflüssigkeitschromatographie (HPLC), der Makrosäule, der Mikrosäule und der - nach Korrektur mit einem gewissen Korrekturfaktor mit der Trennsäulentechnik vergleichbaren - Thiobarbitursäuremethode, Ergebnisse, die sich innerhalb der zu erwartenden systemeigenen Fehlergrenzen bewegen, so daß der Einsatz der erfindungsgemäßen Hämoglobinkontrollen als Richtigkeitskontrolle der zur Bestimmung der Stoffwechsellage von Diabetikern eingesetzten $HbA_1$ Bestimmungsmethoden universelle Bedeutung zukommt.
Auch der mit der jüngst eingeführten affinitätschromatographischen Methode gefundene Wert liegt erwartunsgemäß in derselben Größenordnung.

/8

Der Einsatz der erfindungsgemäßen Hämoglobinstandards als Kontrolle ermöglicht auch mit Trennverfahren, die, wie die Isofokussierung und Elektroosmose aufgrund unterschiedlicher Mechanismen bei der Auftrennung der Hämoglobinfraktionen nicht mit den vorgenannten Verfahren vergleichbar sind, Aussagen, ob das Trennsystem korrekt gehandhabt wurde.

Wie Tabelle 1 zeigt, werden in diesem Fall zwar mit ungefähr 7 % niedrigere Werte für den $HbA_I$-Anteil erhalten, jedoch liegen diese im Bereich der nach diesen Verfahren zu erwartenden Ergebnisse, wenn man von den mit anderen Methoden aufgefundenen Werten ausgeht. Gleiche Verhältnisse werden auch bei Vergleichsmessungen mit Nativblut identischer Zusammensetzung beobachtet.

Die Auftrennung der Gesamtfraktion $HbA_{Iges.}$ in die Einzelfraktionen $HbA_{Ia+b}$ und $HbA_{Ic}$ spielt für die praktische Untersuchung zwar kaum noch eine große Rolle, jedoch können mit der neuen glykosilierten Hämoglobinkontrolle jetzt auch diese Fraktionen unabhängig vom Trennverfahren, wie insbesondere von HPLC, TBA, Makrosäule sowie Isofokussierung auf ihre Richtigkeit überprüft werden.

DR. ING. D. BEHRENS
DIPL. ING. R. GOETZ
PATENTANWÄLTE

TELEFON ... ...
TELEX 524070
TELEGRAMM 0129646
PROTECTPATENT MÜNCHEN

Patentanspruch :

Verfahren zur Herstellung eines glukosefreien Kontroll-Mittels für die interne Kontrolle von spezifischen Trennverfahren für die $HbA_I$-Bestimmung,
dadurch           g e k e n n z e i c h n e t ,
dass man gewaschene Erythrozyten mit destilliertem Wasser oder einschlägigen Reagenzien hämolysiert, im Hämolysat das Hämoglobin mit verdünnter $K_3[Fe(CN)_6]$-Lösung und verdünnter KCN-Lösung in Cyanmethämoglobin überführt, mit einem Puffer stabilisiert und mit einem geeigneten Lösungsmittel Lipide und Zellreste entfernt, worauf man das Hämolysat entsalzt und lyophilisiert.

6029

## EINSCHLÄGIGE DOKUMENTE

EP 84103421.8

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US - A - 3 607 695 (P. SCHNEIDER)<br><br>* Spalte 2, Zeilen 10-45 *<br><br>-- | 1 | G 01 N 33/96<br>G 01 N 33/72 |
| D,A | US - A - 3 964 865 (M.L. DAS)<br><br>* Zusammenfassung *<br><br>---- | 1 | |
| | | | **RECHERCHIERTE SACHGEBIETE (Int. Cl. ³)**<br><br>G 01 N 33/00 |

Der vorliegende Recherchenbericht wurde für alle Patentansprüche erstellt.

| Recherchenort | Abschlußdatum der Recherche | Prüfer |
|---|---|---|
| WIEN | 12-06-1984 | SCHNASS |